# EUROPEAN PATENT APPLICATION

(11) **EP 1 537 866 A1**
(43) Date of publication of application: **08.06.2005**
(21) Application number: 03795352.8
(22) Date of filing: 10.09.2003
(51) Int. Cl.: A61K 31/22, A61P 13/12

(54) **DRUG FOR KIDNEY FAILURE CONTAINING OXALURIC ACID DERIVATIVE**

(30) Priority: 11.09.2002 JP 2002265956
(71) Applicant: Nippon Zoki Pharmaceutical Co. Ltd., Osaka-shi, Osaka 541-0046 (JP)
(72) Inventor: NAIKI, Mitsuru, Nippon Zoki Pharmaceutical Co. Ltd, Kato-gun, Hyogo 673-1461 (JP); NUMAZAWA, Takumi Nippon Zoki Pharmaceutical Co Ltd, Kato-gun, Hyogo 673-1461 (JP)
(74) Representative: HOFFMANN - EITLE
(86) International application number: PCT/JP2003/011542
(87) International publication number: WO 2004/024137

(57) **Abstract**

The effective ingredient of the agent for renal failure of the present invention is an oxaluric acid derivative having an excellent suppressive effect of the progression of renal failure. The oxaluric acid derivatives of the present invention have an excellent suppressive effect of the progression of renal failure to inhibit significantly the increase of creatinine level in blood accompanied by the progression of renal failure. The compounds of the present invention have highly safety with little side effects, so that they are very useful as an agent for renal failure which is required to be administered for long term.

## Description

### Technical Field

The present invention relates to an agent for renal failure containing an oxaluric acid derivative or a pharmaceutically acceptable salt thereof as an effective ingredient.

### Background Art

In Japan, the number of chronic patients treated with hemodialysis is two hundred nineteen thousand (at the end of 2001). The annual increasing number of the patients run up to about thirteen thousand and is further increasing. Great efforts have been given to the prevention, early detection and treatment of renal failure, the suppression for the progression of renal failure, and the supply and spread of medical care of hemodialysis and renal transplantation. The present inventors have carried out investigations for a compound having high safety and being useful as an agent for renal failure, and have consequently found that the oxaluric acid derivatives of the present invention have a suppressive effect of the progression of renal failure whereupon the present invention has been accomplished. It has been known that the oxaluric acid derivatives of the present invention had hypoglycemic effect (Japanese Examined Patent Publication Hei-06/60152). However, regarding the compounds of the present invention, the suppressive effect of the progression of renal failure has not been known at all.

### Disclosure of the Invention

An object of the present invention is to provide a new agent for renal failure with little side effects and high safety. As a result of the intensive investigations for oxaluric acid derivatives, the inventors have found that they have excellent suppressive effects of the progression of renal failure and are very useful as an agent for renal failure whereupon the present invention has been accomplished.

### Brief Description of the Drawing

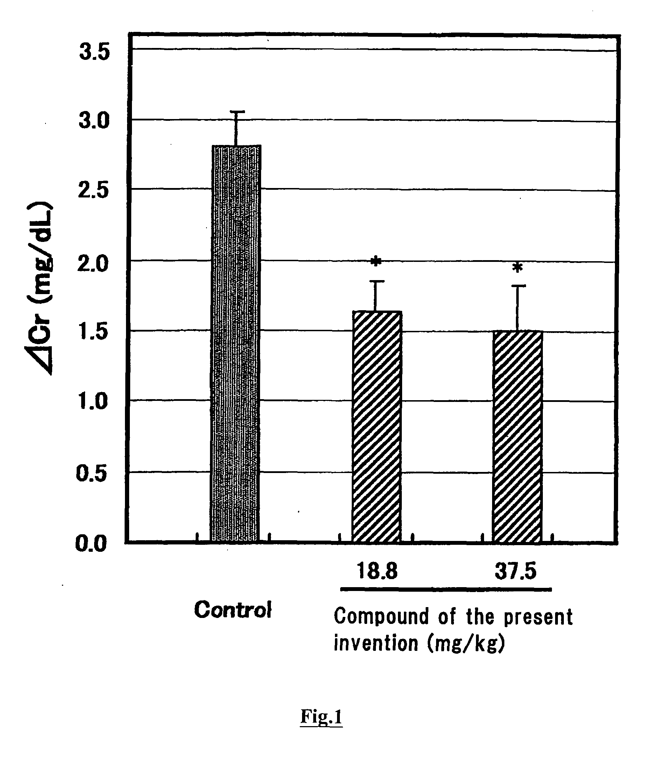

Fig. 1 shows a result studying a suppressive effect of the creatinine increase in blood, when the compound of the present invention, 5-methyloxaluric acid, was administered to model rats of renal failure.

### Best Mode for Carrying Out the Invention

The present invention relates to an agent for renal failure containing at least one oxaluric acid derivative represented by the general formula (I) or a pharmaceutically acceptable salt thereof as an effective ingredient: 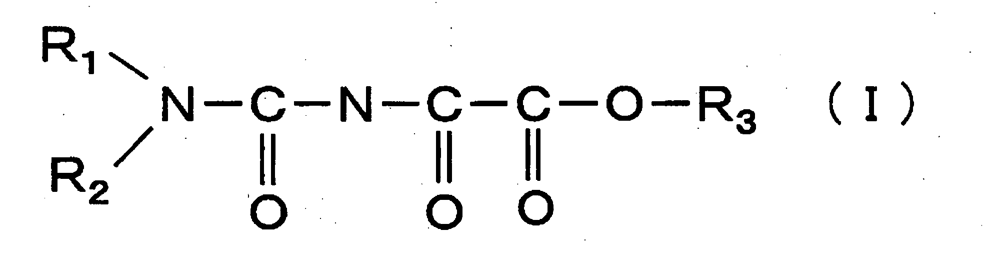 wherein each of R₁ and R₂, which may be the same or different, is hydrogen, an alkyl group or a cycloalkyl group, or R₁ and R₂ are joined to form a heterocyclic ring with the nitrogen atom to which they are both attached, and R₃ is hydrogen or an alkyl group.

In the above mentioned formula (I), each of R₁ and R₂, which may be the same or different, is hydrogen, an alkyl group, preferably a straight or branched alkyl group having 1 to 20 carbon atoms, such as methyl, ethyl, propyl, isopropyl, butyl, isobutyl, sec-butyl, tert-butyl, pentyl, isopentyl, neopentyl, tert-pentyl, hexyl, dimethylbutyl, heptyl, octyl, nonyl, decyl or stearyl; a cycloalkyl group, preferably having 3 to 8 carbon atoms such as cyclopropyl, cyclobutyl, cyclopentyl, cyclohexyl, cycloheptyl or cyclooctyl; or R₁ and R₂ are joined to form a heterocyclic ring with the nitrogen atom they are both attached to, preferably aziridino, pyrrolidino, piperidino, piperazino or morpholino. R₃ is hydrogen or an alkyl group, preferably a straight or branched alkyl group having 1 to 20 carbon atoms, such as methyl, ethyl, propyl, isopropyl, butyl, isobutyl, sec-butyl, tert-butyl, pentyl, isopentyl, neopentyl, tert-pentyl, hexyl, dimethylbutyl, heptyl, octyl, nonyl, decyl or stearyl.

Preferred compounds of the present invention are indicated as follows:
5-methyloxaluric acid
5-ethyloxaluric acid
5-butyloxaluric acid
5-isobutyloxaluric acid
5-tert-butyloxaluric acid
5-hexyloxaluric acid
5-(1,3-dimethylbutyl)oxaluric acid
5-decyloxaluric acid
5-cyclopentyloxaluric acid
5-cyclohexyloxaluric acid
5-methyloxaluric acid ammonium salt
5-ethyloxaluric acid ammonium salt
5-methyloxaluric acid methyl ester
5-methyloxaluric acid ethyl ester
5-butyloxaluric acid methyl ester
5-isobutyloxaluric acid ethyl ester
5,5-dimethyloxaluric acid methyl ester
5,5-dimethyloxaluric acid ethyl ester
5-cyclohexyloxaluric acid ethyl ester
5- cycloheptyloxaluric acid ethyl ester
N-(1-piperidylcarbonyl)oxamic acid methyl ester
N-(1-piperidylcarbonyl)oxamic acid ethyl ester
5-methyloxaluric acid butyl ester
5-methyloxaluric acid isobutyl ester
5-methyloxaluric acid octyl ester
5-butyloxaluric acid butyl ester
5-cyclohexyloxaluric acid isopropyl ester
5-cyclohexyloxaluric acid butyl ester

The oxaluric acid derivatives of the present invention include pharmaceutically acceptable salts of the compounds represented by the above-mentioned formula (I) with alkali metal such as sodium or potassium, with alkaline-earth metal such as calcium, magnesium or barium, with other metal such as aluminium or zinc, with ammonium, with organic amine or with acid. These salts can be prepared from free oxaluric acid derivatives or other salts of the derivatives by known methods.

When there are steric isomers such as cis-trans isomer, optical isomer and conformational isomer, or hydrates and complexes of the compounds of the present invention, the present invention includes any of steric isomers, hydrates and complexes. The compounds of the present invention and the methods for manufacturing them are disclosed in Japanese Examined Patent Publication Hei-06/60152.

The compound of the present invention can be made into pharmaceutical preparations by a combination with a suitable pharmaceutical carriers or diluents according to any conventional methods, for example, preparations for oral administrations (e.g. tablets, capsules, powders, liquids, etc.) and for parenteral administrations (e.g. for subcutaneous, intravenous, intramuscular, intrarectal and intranasal administrations). At preparing, the compound of the present invention may also be used in the form of the pharmaceutically acceptable salt, and can be used either solely or jointly together with other pharmaceutically effective ingredients.

In the case of preparations for oral administration, the compound of the present invention as it is or together with commonly-used excipients such as suitable additives (e.g. lactose, mannitol, corn starch, potato starch, potassium citrate, etc.) is mixed with binders such as cellulose derivatives (e.g. crystalline cellulose, hydroxypropylcellulose, etc.), gum arabicum, corn starch and gelatin, disintegrating agents such as corn starch, potato starch and calcium carboxymethylcellulose, lubricants such as talc and magnesium stearate and other agents such as bulking agents, moisturizing agents, buffers, preservatives, perfumes and the like to give tablets, powders, granules or capsules.

In the case of injections, it is possible to prepare the solution, suspension or emulsion in aqueous solvents such as distilled water for injection, physiological saline solution and glucose solution for injection, or non-aqueous solvents such as plant oil, synthetic fatty acid glycerides, higher fatty acid esters and propylene glycol. If necessary, conventional excipients such as solubilizing agents, isotonizing agents, suspending agents, emulsifiers, stabilizers and preservatives may be added.

Furthermore, depending upon the type of the disease and patient, it is possible to prepare other preparations than those which were mentioned already, for example, suitable preparations for the therapy, such as suppositories, inhalations, aerosols, syrups, collyriums, medicines for external use (e.g. ointments), etc.

The preferred dose of the compound of the present invention may vary depending upon the patient to be administered, the preparation form, the method and term of the administration, etc. In order to achieve a desired effect, 50-5,000 mg per day, preferably 100-3,000 mg per day may be usually given to common adults by oral route. In the case of parenteral administration such as by injection, since the effect may be expected at parenteral administration by smaller dose than that of oral administration, a dosage level of from 1/3 to 1/10 of the above given dose by oral administration may give the desired effect.

Preferred embodiments of the agent for renal failure containing the compound represented by the above formula (I) as an effective ingredient are given as follows.
(1) An agent for renal failure containing the compound represented by the formula (I), wherein R₃ represents hydrogen, as an effective ingredient.
(2) The agent according to the above (1) containing the compounds, wherein R₂ represents hydrogen, as an effective ingredient.
(3) The agent according to the above (2) containing the compounds, wherein R₁ represents an alkyl group, as an effective ingredient.
(4) The agent according to the above (3) containing the compounds, wherein R₁ represents methyl, as an effective ingredient.
(5) The agent for renal failure containing 5-methyloxaluric acid or a pharmaceutically acceptable salt thereof as an effective ingredient.
(6) The suppressive agent of the progression of renal failure containing 5-methyloxaluric acid or a pharmaceutically acceptable salt thereof as an effective ingredient.

### Example 1 : Suppressive effect of the progression of renal failure

Model animals of renal failure were prepared by oral administration of adenine to male Wistar-strain rats (8 weeks age) [cf. Kidney and Dialysis (extra number), p.440-445 (1991); Nephron, vol.44, p.230-234 (1986), etc.]. By daily administrations of adenine (200mg/kg), the creatinine level in blood as an indicator of renal function was gradually increased from 0.44±0.02 mg/dL before administration to 4.12±0.53 mg/dL after about 3 weeks. The creatinine clearance values (mL/kg/hr) were decreased from 315.3±13.8 to 25.7±4.4, and thus the renal function fell to about 1/10.

The compound of the present invention, 5-methyloxaluric acid, was orally administered to the adenine-induced renal failure rat at the dose of 18.8 mg/kg or 37.5 mg/kg daily for 17 days from the 7th day after the administration of adenine. The creatinine levels in blood were compared between before and after administration of the test drug. The result of the suppressive effect of the test drug against creatinine increase (Δ Cr) accompanied by the progression of renal failure is shown in Fig. 1. Five rats were used as a group for the test. The average value ± standard error was calculated and the significant difference between the test drug group and the diseased control group was statistically analyzed by means of Dunnett's test (*; p<0.05).

### Example 2 : Single dose toxicity test

5-Methyloxaluric acid was intravenously administered to SD-strain rats (6 weeks age, 5 males and 5 females) at the dose of 75 to 600 mg/kg. As a result, even at the dose of 600 mg/kg, any abnormalities of general symptoms, body weight and autopsies are observed and, as a matter of course, there is no death.

### Industrial Applicability

It is apparent from the above-mentioned pharmacological tests that the compounds of the present invention significantly suppressed the increase of creatinine levels in blood accompanied by the progression of renal failure. Moreover, no toxicities were observed in the single dose toxicity test using rats. As above-mentioned, the compounds of the present invention exhibit an excellent suppressive effect of the progression of renal failure and have high safety with little side effects, so that they are very useful as an agent for renal failure which is required to be administered for long term.

## Claims

1. An agent for renal failure containing at least one oxaluric acid derivative represented by the following formula (I) or a pharmaceutically acceptable salt thereof as an effective ingredient. 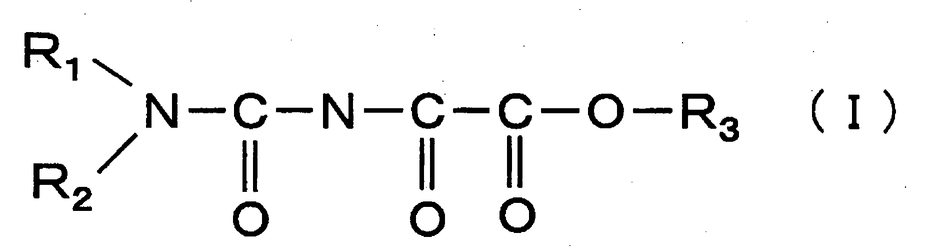 wherein each of R₁ and R₂, which may be the same or different, is hydrogen, an alkyl group or a cycloalkyl group, or R₁ and R₂ are joined to form a heterocyclic ring with the nitrogen atom to which they are both attached, and R₃ is hydrogen or an alkyl group.

2. The agent for renal failure according to claim 1, wherein the agent is therapeutic or preventive agent for renal failure.

3. The agent for renal failure according to claim 2, wherein the agent is therapeutic or preventive agent for acute renal failure.

4. The agent for renal failure according to claim 2, wherein the agent is therapeutic or preventive agent for chronic renal failure.

5. An agent for renal failure according to claim 1, wherein the suppressive agent for the progression of renal failure.
